# EUROPÄISCHE PATENTSCHRIFT

(11) **EP 1 106 169 B1**
(45) Veröffentlichungstag und Bekanntmachung des Hinweises auf die Patenterteilung: **01.06.2005**
(21) Anmeldenummer: 00125866.4
(22) Anmeldetag: 25.11.2000
(51) Int. Cl.: A61K 7/48, A61K 7/42

(54) **Emulsionen**
Emulsions
Emulsions

(30) Priorität: 08.12.1999 DE 19959119
(43) Veröffentlichungstag der Anmeldung: 13.06.2001
(73) Patentinhaber: Clariant GmbH, 65929 Frankfurt am Main (DE)
(72) Erfinder: Löffler, Matthias, Dr., 65527 Niedernhausen (DE)

(56) Entgegenhaltungen:
- EP-A- 0 673 638
- FR-A- 2 760 643
- FR-A- 2 781 233
- US-A- 4 785 060
- CHEMICAL ABSTRACTS, vol. 130, no. 1, 4. Januar 1999 (1999-01-04) Columbus, Ohio, US; abstract no. 5129, "Optimised soil release polymers. Basic effects, detergency benefits, and interactions with detergent ingredients" XP002169316 & LANG,FRANK-PETER: CHIM. OGGI, Bd. 16, Nr. 9, 1998, Seiten 14-18,

## Beschreibung

Es ist bekannt, dass Oligoester als sogenannte Schmutzablösepolymere (Soil Release Polymers) in Wasch- und Reinigungsmitteln eingesetzt werden. Bei diesen Oligoestern handelt es sich um Kondensationsprodukte aus Dimethylterephthalat, Ethylenglykol, Propylenglykol und Polyalkylenglykolen. Infolge eines molaren Überschusses der Alkoholkomponente enthalten diese Oligoester endständige OH-Gruppen, die ganz oder teilweise durch Alkoxygruppen abgeschlossen sein können (end-caps). Verbindungen dieser Art sind unter den Bezeichnungen ®Milease T, ®Permalose, ®Repel-O-Tex kommerziell erhältlich. Bezüglich näherer Einzelheiten sei auf die Schriften EP 185 427, EP 241 984, EP 241 985, EP 272 033, EP 757 468, EP 201 124, EP 253 567, GB 2 304 727, US 4 116 885, US 4 210 417, US 3 962 152, WO 92/17 523, verwiesen.

Es wurde nun gefunden, dass sich derartige Oligoester überraschenderweise auch hervorragend als Emulgator für Emulsionen eignen.

Gegenstand der Erfindung ist somit die Verwendung von Oligoestern als Emulgator für Emulsionen.

Diese Oligoester werden vorzugsweise erhalten durch Polykondensation von einer oder mehreren aromatischen Dicarbonsäuren oder deren Ester und einem oder mehreren mehrwertigen Alkoholen wie beispielsweise Ethylenglykol und/oder Propylenglykol. Gegebenenfalls können diese Ester auch Polyethylenglykol, Polypropylenglykol, Sulfoisophthalsäure, Sulfobenzoesäure, Isethionsäure, C₁-C₄-Alkohole, oxalkylierte C₁-C₂₄-Alkohole, oxalkylierte C₆-C₁₈-Alkylphenole und/oder oxalkylierte C₈-C₂₄-Alkylamine als Monomere enthalten.

Insbesondere bevorzugt sind Emulsionen mit solchen Oligoestern, die erhalten wurden durch Polykondensation von
a) 40 bis 52, vorzugsweise 45 bis 50 mol% einer oder mehrerer Dicarbonsäuren oder deren Ester,
b) 10 bis 60, vorzugsweise 20 bis 35 mol% Ethylenglykol und/oder Propylenglykol,
c) 0 bis 20, vorzugsweise 10 bis 15 mol% Polyethylenglykol,
d) 0 bis 10 mol% eines wasserlöslichen Anlagerungsproduktes von 5 bis 80 mol eines Alkylenoxids an 1 mol C₁-C₂₄-Alkohole, C₆-C₁₈-Alkylphenole oder C₈-C₂₄-Alkylamine und
e) 0 bis 10 mol% eines oder mehrerer Polyole mit 3 bis 6 Hydroxylgruppen.

Als Komponente a) zur Herstellung der Oligoester eignen sich aromatische Dicarbonsäuren wie beispielsweise Terephthalsäure, Phthalsäure, Isophthalsäure sowie die Mono- und Dialkylester mit C₁- bis C₆-Alkoholen, wie Dimethylterephthalat, Diethylterephthalat und Di-n-propylterephthalat. Weitere Beispiele für Verbindungen, die als Komponente a) zur Herstellung der Polyester eingesetzt werden können, sind Oxalsäure, Bernsteinsäure, Glutarsäure, Adipinsäure, Fumarsäure, Maleinsäure, Itakonsäure, sowie die Mono- und Dialkylester der Carbonsäuren mit C₁-C₆-Alkoholen, z.B. Oxalsäurediethylester, Bemsteinsäurediethylester, Glutarsäurediethylester, Adipinsäuremethylester, Adipinsäurediethylester, Adipinsäure-di-n-butylester, Fumarsäureethylester und Maleinsäuredimethylester. Sofern die in Betracht kommenden Dicarbonsäuren Anhydride bilden können, sind auch die Anhydride der mindestens 2 Carboxylgruppen aufweisenden Carbonsäuren als Verbindung der Komponente a) zur Herstellung der Oligoester geeignet, z.B. Maleinsäureanhydrid, Phthalsäureanhydrid oder Bernsteinsäureanhydrid. Besonders bevorzugt werden als Verbindung der Komponente a) Terephthalsäure, Phthalsäure, Isophthalsäure sowie deren Dimethyl-, Diethyl-, Dipropyl- und Dibutylester eingesetzt. Es ist selbstverständlich möglich Mischungen verschiedener Carbonsäuren oder verschiedener Ester einzusetzen. Ebenso kann man auch beispielsweise Mischungen aus Carbonsäuren und Estern oder Mischungen aus Carbonsäuren und Anhydriden bei der Kondensation verwenden.

Als Komponente c) verwendet man Polyethylenglykole mit Molmassen von 500 bis 5000, vorzugsweise von 1000 bis 3000.

Als Komponente d) zur Herstellung der Oligoester kommen wasserlösliche Anlagerungsprodukte von 5 bis 80 mol mindestens eines Alkylenoxids an 1 mol C₁-C₂₄-Alkohole, C₆-C₁₈-Alkylphenole oder C₈-C₂₄-Alkylamine in Betracht. Bevorzugt sind Monomethylether von Polyethylenglykolen. Als Alkylenoxide zur Herstellung der Verbindungen der Komponente d) verwendet man vorzugsweise Ethylenoxid, sowie Mischungen aus Ethylenoxid und Propylenoxid. Außerdem eignen sich Mischungen aus Ethylenoxid zusammen mit Propylenoxid und/oder Butylenoxid, Mischungen aus Ethylenoxid, Propylenoxid und Isobutylenoxid oder Mischungen aus Ethylenoxid und mindestens einem Butylenoxid. Diese wasserlöslichen Anlagerungsprodukte der Alkylenoxide sind Tenside. Falls zu ihrer Herstellung Mischungen von Alkylenoxiden verwendet werden, so können sie die Alkylenoxide in Blöcken oder auch in statistischer Verteilung enthalten.

Geeignete Alkohole, die alkoxyliert werden, sind beispielsweise Octylalkohol, Decylalkohol, Laurylalkohol, Myristylalkohol oder Stearylalkohol, insbesondere aber Methanol, sowie die nach dem Ziegler-Verfahren erhältlichen Alkohole mit 8 bis 24 C-Atomen oder die entsprechenden Oxoalkohole. Von den Alkylphenolen sind insbesondere Octylphenol, Nonylphenol und Dodecylphenol von Bedeutung. Als Alkylamine verwendet man insbesondere die C₁₂-C₁₈-Monoalkylamine.

Als Polyole (Komponente e) kommen beispielsweise Pentaerythrit, Trimethylolethan, Trimethylolpropan, 1,2,3-Hexantriol, Sorbit, Mannit und Glycerin in Frage.

Die Synthese der erfindungsgemäßen Oligoester erfolgt nach an sich bekannten Verfahren, indem die Komponenten a, b und c sowie gegebenenfalls d unter Zusatz eines Katalysators zunächst bei Normaldruck auf Temperaturen von 160 bis ca. 220°C erhitzt werden. Dann wird die Reaktion im Vakuum bei Temperaturen von 160 bis ca. 240°C unter Abdestillieren überschüssiger Glykole fortgesetzt. Für die Reaktion eignen sich die bekannten Umesterungs- und Kondensationskatalysatoren des Standes der Technik, wie beispielsweise Titantetraisopropylat, Dibutylzinnoxid oder Antimontrioxid/Calciumacetat. Bezüglich weiterer Einzelheiten zur Durchführung des Verfahrens wird auf EP 442 101 verwiesen.

Besonders geeignet sind auch die aus EP 241 985 bekannten Polyester, die neben Oxyethylen-Gruppen und Terephthalsäureeinheiten 1,2-Propylen-, 1,2-Butylenund/oder 3-Methoxy-1,2-propylengruppen sowie Glycerineinheiten enthalten und mit C₁- bis C₄-Alkylgruppen endgruppenverschlossen sind; die in EP 253 567 beschriebenen schmutzablösevermögenden Polymere mit einer Molmasse von 900 bis 9000 g/mol aus Ethylenterephthalat und Polyethylenoxidterephthalat, wobei die Polyethylenglykol-Einheiten Molgewichte von 300 bis 3000 g/mol aufweisen und das Molverhältnis von Ethylenterephthalat zu Polyethylenoxidterephthalat 0,6 bis 0,95 beträgt; und die aus EP 272 033 bekannten, zumindest anteilig durch C₁-C₄-Alkylreste endgruppenverschlossenen, Polyester mit Polypropylenterephthalat- und Polyoxyethylenterephthalat Einheiten.

Gleichfalls bevorzugt sind Oligoester aus Ethylenterephthalat und Polyethylenoxidterephthalat, in denen die Polyethylenglykol-Einheiten Molgewichte von 750 bis 5000 g/mol aufweisen und das Molverhältnis von Ethylenterephthalat zu Polyethylenoxidterephthalat 50:50 bis 90:10 beträgt und deren Einsatz in Waschmitteln in der deutschen Patentschrift DE 28 57 292 beschrieben ist, sowie Oligoester mit Molgewichten von 15 000 bis 50 000 g/mol aus Ethylenterephthalat und Polyethylenoxidterephthalat, wobei die Polyethylenglykol-Einheiten Molgewichte von 1000 bis 10 000 g/mol aufweisen und das Molverhältnis von Ethylenterephthalat zu Polyethylenoxidterephthalat 2:1 bis 6:1 beträgt, die gemäß DE 33 24 258 in Waschmitteln eingesetzt werden können.

Ebenfalls bevorzugt sind die in DE 19 644 034 beschriebenen Oligoester der Formel 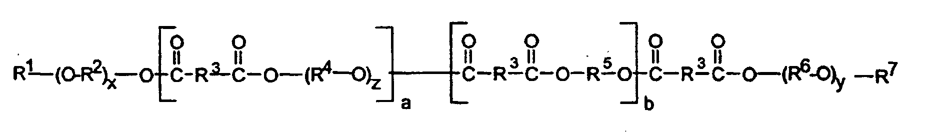 worin
- R¹ und R⁷: lineares oder verzweigtes C₁- bis C₁₈-Alkyl,
- R² und R⁶: Ethylen,
- R³: 1,4-Phenylen,
- R⁴: Ethylen,
- R⁵: Ethylen, 1,2-Propylen oder statistische Gemische von beliebiger Zusammensetzung von beiden,
- x und y: unabhängig voneinander eine Zahl zwischen 1 und 500,
- z: eine Zahl zwischen 10 und 140,
- a: eine Zahl zwischen 1 und 12,
- b: eine Zahl zwischen 7 und 40,
bedeuten, wobei a+b mindestens gleich 11 ist.

Bevorzugt bedeuten unabhängig voneinander
- R¹ und R⁷: lineares oder verzweigtes C₁- bis C₄-Alkyl,
- x und y: eine Zahl zwischen 3 und 45,
- z: eine Zahl zwischen 18 und 70,
- a: eine Zahl zwischen 2 und 5,
- b: eine Zahl zwischen 8 und 12 und
a+b eine Zahl zwischen 12 und 18 oder zwischen 25 und 35. Die in DE 19 644 034 beschriebenen Oligoester werden aus Dimethylterephthalat, Ethylen- und/oder Propylenglykol, Polyethylenglykol und C₁- bis C₁₈-Alkylpolyethylenglykol unter Zusatz eines Katalysators zunächst durch Umesterung bei Temperaturen von 160 bis ca. 220°C und destillativer Abtrennung des Methanols bei Normaldruck und anschließender destillativer Abtrennung der überschüssigen Glykole bei Temperaturen von 160 bis ca. 240°C erhalten.

Die beschriebenen Oligoester sind in den erfindungsgemäßen Emulsionen üblicherweise in Mengen von 0,1 bis 5, vorzugsweise 0,3 bis 3 Gewichtsprozent, bezogen auf die fertige Emulsion, enthalten. Die Emulsionen können sowohl Wasser-in-Öl-Emulsionen als auch Öl-in-Wasser-Emulsionen sein.

Der nichtwässrige Anteil der Emulsionen, der sich weitgehend aus dem Emulgatorund dem Ölkörper zusammensetzt und dabei in der Regel dem Feststoffgehalt entspricht, liegt üblicherweise bei 5 bis 95 % und vorzugsweise 15 bis 75 Gew.-%.

Das bedeutet, dass die Emulsionen 5 bis 95 und vorzugsweise 25 bis 85 Gew.-% Wasser enthalten können, abhängig davon, ob Lotionen mit einer vergleichsweise niedrigen oder Cremes und Salben mit hoher Viskosität hergestellt werden sollen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₁₃ Fettsäuren mit linearen C₆-C₂₀ Fettalkoholen, Ester von verzweigten C₆-C₁₃ Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. aromatische Kohlenwasserstoffe in Betracht.

Die Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe, Co-Emulgatoren, Überfettungsmittel, Fette, Wachse, Stabilisatoren, biogene Wirkstoffe, Glycerin, Konservierungsmittel, Farb- und Duftstoffe enthalten.

Wesentlich für die Erfindung ist, dass die beschriebenen Oligoester auch ohne Mitverwendung eines nicht-ionischen Tensids als Co-Emulgator eingesetzt werden können. Die Mitverwendung von Co-Emulgatoren ist daher nicht zwingend, aber möglich.

Als nichtionogene O/W-Co-Emulgatoren kommen in Betracht Anlagerungsprodukte von 2 bis 30 Mol Ethylenoxid und/oder 0 bis 5 Mol Propylenoxid an lineare Fettalkohole mit 8 bis 22 Atomen, an Fettsäuren mit 12 bis 22 C-Atomen und an Alkylphenole mit 8 bis 15 C-Atomen in der Alkylgruppe; C₁₂-C₁₈-Fettsäuremono- und -diester von Anlagerungsprodukten von 1 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester wie z.B. Polyglycerinpolyricinoleat und Polyglycerinpoly-12-hydroxystearat. Ebenfalls geeignet sind Gemische von Verbindungen aus mehreren dieser Substanzklassen. Die Anlagerungsprodukte von Ethylenoxid und/oder Propylenoxid an Fettalkohole, Fettsäuren, Alkylphenole, Glycerin-mono- und -diester sowie Sorbitanmono- und -diester von Fettsäuren oder an Rizinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht C₁₂-C₁₈-Fettsäuremono- und -diester von Anlagerungsprodukten von Ethylenoxid an Glycerin sind aus DE 20 24 051 als Rückfettungsmittel für kosmetische Zubereitungen bekannt.

Als Überfettungsmittel können Substanzen wie beispielsweise polyethoxilierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage. Als Stabilisatoren können Metallsalze von Fettsäuren wie z.B. Magnesium-, Aluminium- und/oder Zinkstearat eingesetzt werden. Unter biogenen Wirkstoffen sind beispielsweise Pflanzenextrakte und Vitaminkomplexe zu verstehen. Als Konservierungsmittel eignen sich beispielsweise Phenoxyethanol, Formaldehydlösung, Parabene, Pentandiol oder Sorbinsäure. Als Perlglanzmittel kommen beispielsweise Glycoldistearinsäureester wie Ethylenglycoldistearat, aber auch Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind.
Der Gesamtanteil der Hilfs- und Zusatzstoffe kann 1 bis 10, vorzugsweise 2 bis 5 Gew.-% bezogen auf das Mittel, betragen.

Die Herstellung der Mittel kann in an sich bekannter Weise, d.h. beispielsweise durch Heiß-, Heiß-Heiß/ Kalt- bzw. PIT- Emulgierung erfolgen.

Mit dem erfindungsgemäßen Einsatz der Oligoester in Emulsionen, insbesondere in O/W Lotionen und O/W Cremes, lassen sich stabile Formulierungen erhalten, die zudem ein angenehmes Hautgefühl vermitteln.

Die nachfolgenden Beispiele sollen den Gegenstand der Erfindung näher erläutern,

Folgende erfindungsgemäßen Oligoester wurden in den Beispielen verwendet:
- Polyester 1:: 40 mol% Terephthalsäure, 10 mol% Ethylenglykol, 10 mol% Propylenglykol, 20 mol% Polyethylenglykol, 10 mol% Fettalkoholethoxylat, 10 mol% Polyol
- Polyester 2:: 50 mol% Terephthalsäure, 25 mol% Ethylenglykol, 20 mol% Polyethylenglykol, 5 mol% Fettalkoholethoxylat
- Polyester 3:: 50 mol% Hexandicarbonsäure, 40 mol% Propylenglycol, 10 mol% Polyethylenglykol

Die Herstellung dieser Polyester erfolgte in der eingangs beschriebenen Weise. Folgende Stabilitätstests wurden mit den nachfolgend beschriebenen Formulierungen durchgeführt:
Lagerung bei 40°C, 45°C und 50°C über einen Zeitraum von 90 Tagen. Zentrifugieren bei 20°C, 5000 U/min, 30 min

Alle Formulierungen zeigten unter diesen Bedingungen eine gute bis sehr gute Stabilität.

### Beispiel 1

| O/W Creme | | | |
|---|---|---|---|
| A | POLYESTER 1 | (Clariant) | 1,00% |
| | ®Cetiol V | | 7,00% |
| | Jojoba oil | | 5,00% |
| | Isopropylpalmitat | | 6,00% |
| B | ®Aristoflex AVC | (Clariant) | 0,70% |
| C | Glycerin | | 3,00% |
| | Wasser | | 76,90% |
| | Konservierungsmittel | | q. s. |
| D | Parfüm | | 0,40% |

### Herstellung

I B wurde in A eingerührt, dann wurde C zugegeben und gut gerührt
II Unter Rühren wurde D zugegeben
III Die Emulsion wurde homogenisiert.

### Beispiel 2

| O/W Creme | | | |
|---|---|---|---|
| A | POLYESTER 2 | (Clariant) | 1,00% |
| | ®Cetiol V | | 7,00% |
| | Jojoba oil | | 5,00% |
| | Isopropylpalmitat | | 6,00% |
| B | ®Aristoflex AVC | (Clariant) | 0,70% |
| C | Glycerin | | 3,00% |
| | Wasser | | 76,90% |
| | Konservierungsmittel | | q. s. |
| D | Parfüm | | 0,40% |

### Herstellung: wie in Beispiel 1

### Beispiel 3

| O/W Creme | | | |
|---|---|---|---|
| A | POLYESTER 1 | (Clariant) | 1,50% |
| | Mineralöl, niederviskos | | 8,00% |
| | Isopropylpalmitat | | 4,00% |
| | ®Eutanol G | | 4,00% |
| B | ®Aristoflex AVC | (Clariant) | 0,70% |
| C | Wasser | | 81,40% |
| | Konservierungsmittel | | q. s. |
| D | Parfüm | | 0,40% |

### Herstellung: wie in Beispiel 1

### Beispiel 4

| O/W Creme | | | |
|---|---|---|---|
| A | POLYESTER 2 | (Clariant) | 1,00% |
| | Mineralöl, niederviskos | | 8,00% |
| | Isopropylpalmitat | | 4,00% |
| | ®Eutanol G | | 4,00% |
| B | ®Aristoflex AVC | (Clariant) | 0,70% |
| C | Wasser | | 81,90% |
| | Konservierungsmittel | | q. s. |
| D | Parfüm | | 0,40% |

### Herstellung: wie in Beispiel 1

### Beispiel 5

| O/W Sonnenschutzmilch | | | |
|---|---|---|---|
| A | POLYESTER 1 | (Clariant) | 1,00% |
| | Mineralöl, hoch viskos | | 10,00% |
| | Isopropylpalmitat | | 5,00% |
| B | ®Neo - Heliopan E 1000 | | 8,50% |
| | ®Neo - Heliopan BB | | 1,50% |
| C | Aristoflex AVC | (Clariant) | 0,60% |
| D | Glycerin | | 3,00% |
| | Wasser | | 70,10% |
| | Konservierungsmittel | | q. s. |
| E | Parfüm | | 0,30% |

### Herstellung:

I B wurde mit A gemischt, dann wurde C zugegeben
II D wurde unter Rühren zur Mischung I gegeben, dann wurde E zugegeben,
III Die Emulsion wurde homogenisiert

### Beispiel 6

| O/W Sonnenschutzmilch | | | |
|---|---|---|---|
| A | POLYESTER 2 | (Clariant) | 1,00% |
| | Mineralöl, hoch viskos | | 10,00% |
| | Isopropylpalmitat | | 5,00% |
| B | ®Neo ― Heliopan E 1000 | | 8,50% |
| | ®Neo ― Heliopan BB | | 1,50% |
| C | Aristoflex AVC | (Clariant) | 0,60% |
| D | Glycerin | | 3,00% |
| | Wasser | | 70,10% |
| | Konservierungsmittel | | q. s. |
| E | Parfüm | | 0,30% |

### Herstellung: wie in Beispiel 5

### Beispiel 7

| O/W Sonnenschutzmilch | | | |
|---|---|---|---|
| A | POLYESTER 3 | (Clariant) | 2,00% |
| | Mineralöl, hoch viskos | | 10,00% |
| | Isopropylpalmitat | | 5,00% |
| B | ®Neo ― Heliopan E 1000 | | 8,50% |
| | ®Neo ― Heliopan BB | | 1,50% |
| C | Aristoflex AVC | (Clariant) | 0,60% |
| D | Glycerin | | 3,00% |
| | Wasser | | 69,10% |
| | Konservierungsmittel | | q. s. |
| E | Parfüm | | 0,30% |

### Herstellung: wie Beispiel 5

Chemische Bezeichnung der eingesetzten Handelsprodukte:
- Aristoflex AVC:: Acrylamidopropylensulfonsäure/Vinylformamid-Copolymer
- Cetiol:: Decyloleat
- Eutanol G:: Hexyldecanol
- Neo-Heliopan E 1000:: Isoamyl-p-methoxycinnamate
- Neo-Heliopan BB:: Benzophenone-3

## Patentansprüche

1. Verwendung von Oligoestern, die durch Polykondensation von einer oder mehreren Dicarbonsäuren oder deren Ester und Ethylenglykol und/oder Propylenglykol, sowie gegebenenfalls Polypropylenglykol, Polyethylenglykol, Sulfoisophthalsäure, Sulfobenzoesäure, Isethionsäure, C₁-C₄-Alkoholen, oxalkylierten C₁-C₂₄-Alkoholen, oxalkylierten C₆-C₁₈-Alkylphenolen oder oxalkylierten C₈-C₂₄-Alkylaminen erhalten werden, als Emulgator für Emulsionen, deren nichtwässriger Anteil bei 5 bis 95 Gew.-% liegt.

2. Verwendung nach Anspruch 1, **dadurch gekennzeichnet, dass** die Oligoester durch Polykondensation von
a) 40 bis 52, vorzugsweise 45 bis 50 mol%, einer oder mehrerer aromatischer Dicarbonsäuren oder deren Ester,
b) 10 bis 60, vorzugsweise 20 bis 35 mol-%, Ethylenglykol und/oder Propylenglykol,
c) 0 bis 20, vorzugsweise 10 bis 15 mol%, Polyethylenglykol,
d) 0 bis 10 mol% eines wasserlöslichen Anlagerungsproduktes von 5 bis 80 mol eines Alkylenoxids an 1 mol C₁-C₂₄-Alkohole, C₆-C₁₈-Alkylphenole oder C₈-C₂₄-Alkylamine und
e) 0 bis 10 mol% eines oder mehrerer Polyole mit 3 bis 6 Hydroxylgruppen erhalten werden.

3. Verwendung nach Anspruch 1, **dadurch gekennzeichnet, dass** die Emulsionen 0,1 bis 5 Gew.-% Oligoester enthalten.

## Claims

1. The use of oligoesters obtained by polycondensation of one or more dicarboxylic acids or esters thereof and ethylene glycol and/or propylene glycol, and optionally polypropylene glycol, polyethylene glycol, sulfoisophthalic acid, sulfobenzoic acid, isethionic acid, C₁-C₄-alcohols, oxalkylated C₁-C₂₄-alcohols, oxalkylated C₆-C₁₈-alkylphenols or oxalkylated C₈-C₂₄-alkylamines as emulsifier for emulsions having a nonaqueous fraction of 5 to 95%.

2. The use according to claim 1, **characterized in that** the oligoesters are obtained by polycondensation of
a) 40 to 52 mol%, preferably 45 to 50 mol%, of one or more aromatic dicarboxylic acids or esters thereof,
b) 10 to 60 mol%, preferably 20 to 35 mol%, of ethylene glycol and/or propylene glycol,
c) 0 to 20 mol%, preferably 10 to 15 mol%, of polyethylene glycol,
d) 0 to 10 mol% of a water-soluble addition product of from 5 to 80 mol of an alkylene oxide with 1 mol of C₁-C₂₄-alcohols, C₆-C₁₈-alkylphenols or C₈-C₂₄-alkylamines and
e) 0 to 10 mol% of one or more polyols having 3 to 6 hydroxyl groups.

3. The use according to claim 1, **characterized in that** the emulsions comprise 0.1 to 5% by weight of oligoesters.

## Revendications

1. Utilisation d'oligoesters que l'on obtient par polycondensation d'un ou plusieurs acides dicarboxyliques ou esters de ceux-ci avec de l'éthylèneglycol et/ou du propylèneglycol, ainsi qu'avec, éventuellement, du polypropylèneglycol, du polyethylèneglycol, de l'acide sulfoisophtalique, de l'acide sulfobenzoïque, de l'acide iséthionique, des alcools en C₁ à C₄, des alcools en C₁ à C₂₄ oxalkylés, des (alkyle en C₆ à C₁₈)phénols oxalkylés ou des (alkyle en C₈ à C₂₄)amines oxalkylées, en tant qu'émulsifiants pour des émulsions dont la phase non aqueuse représente de 5% à 95% en poids.

2. Utilisation selon la revendication 1, **caractérisée en ce que** l'on obtient les oligoesters par polycondensation de :
a) 40% à 52%, de préférence 45% à 50% en moles, d'un ou plusieurs acides dicarboxyliques aromatiques ou esters de ceux-ci,
b) 10% à 60%, de préférence 20% à 35% en moles, d'éthylèneglycol et/ou de propylèneglycol,
c) 0% à 20%, de préférence 10% à 15% en moles, de polyéthylèneglycol,
d) 0% à 10% en moles d'un produit d'addition hydrosoluble de 5 à 80 moles d'un oxyde d'alkylène à 1 mole d'alcools en C₁ à C₂₄, d'(alkyle en C₆ à C₁₈)phénols ou d'(alkyle en C₈ à C₂₄)amines et
e) 0% à 10% en moles d'un ou plusieurs polyols comprenant 3 à 6 groupes hydroxyle.

3. Utilisation selon la revendication 1, **caractérisée en ce que** les émulsions contiennent 0,1% à 5% en poids d'oligoesters.
